# FASCICULE DE BREVET EUROPEEN

(11) **EP 0 780 113 B1**
(45) Date de publication et mention de la délivrance du brevet: **04.09.2002**
(21) Numéro de dépôt: 96402526.6
(22) Date de dépôt: 22.11.1996
(51) Int. Cl.: A61K 7/00, A61K 7/48

(54) **Dispersion stable d'une phase non miscible à l'eau, dans une phase aqueuse au moyen de vésicules à base de tensioactif silicone**
Stabile Dispersion einer nicht mit Wasser mischbaren Phase, in einer wässrigen Phase mittels tensioaktiven Silikonvesikel
Stable dispersion of a phase not miscable in water, in an aqueous phase using tensioactive siliconvesicles

(30) Priorité: 21.12.1995 FR 9515292
(43) Date de publication de la demande: 25.06.1997
(73) Titulaire: L'OREAL, 75008 Paris (FR)
(72) Inventeur: Simonnet, Jean-Thierry, 75011 Paris (FR)
(74) Mandataire: Tezier Herman, Béatrice

(56) Documents cités:
- EP-A- 0 043 327
- EP-A- 0 226 337
- EP-A- 0 330 369
- EP-A- 0 407 089
- EP-A- 0 444 983
- EP-A- 0 514 934
- EP-A- 0 526 289
- EP-A- 0 529 847
- EP-A- 0 559 013
- EP-A- 0 579 455
- EP-A- 0 631 774
- EP-A- 0 638 308
- FR-A- 2 315 991
- FR-A- 2 597 345
- FR-A- 2 597 367
- FR-A- 2 683 453
- FR-A- 2 693 466
- US-A- 5 364 633
- TENSIDE,SURFACTANT,DETERGENTS, vol. 29, no. 2, Mars 1992, MÜNICH, pages 78-83, XP000262468 B. GRÜNING ET AL:

## Description

La présente invention se rapporte à une dispersion d'une phase non miscible à l'eau dans une phase aqueuse externe ou continue au moyen de vésicules lipidiques comportant un tensioactif siliconé, et à son utilisation pour le traitement de la peau, des muqueuses, des ongles, du cuir chevelu et/ou des cheveux.

On entend par vésicules lipidiques des particules formées d'une phase lamellaire constituée par un ou plusieurs feuillets concentriques, ces feuillets comportant une ou plusieurs couches bimoléculaires de lipides amphiphiles encapsulant une phase aqueuse. La phase aqueuse encapsulée peut contenir des substances actives hydrosolubles et les couches bimoléculaires de lipides amphiphiles peuvent contenir des substances actives lipophiles.

Il est connu dans les domaines cosmétique et dermatologique d'appliquer sur la peau des compositions sous forme de dispersions aqueuses contenant une phase non miscible à l'eau et des vésicules lipidiques assurant la dispersion de la phase non miscible à l'eau dans la phase aqueuse.

Ainsi, les documents FR-A-2490504 et FR-A-2485921 décrivent la stabilisation d'une dispersion de gouttelettes d'un liquide non miscible à l'eau et notamment d'huile, au moyen de vésicules lipidiques sans qu'il soit nécessaire d'introduire dans la dispersion un autre agent de stabilisation et notamment un émulsionnant. Les vésicules décrites dans ces documents sont obtenues à partir de lipides ioniques ou non ioniques. Toutefois, ces dispersions présentent l'inconvénient de donner une texture collante, ce qui rend leur utilisation peu agréable. On pallie à cet inconvénient par le choix des huiles.

Par ailleurs, pour obtenir une structure suffisamment épaisse, on ajoute généralement des gélifiants à ces dispersions d'huile. Malheureusement, la nature de ces vésicules limite la quantité de gélifiant à utiliser et donc limite la consistance de la composition.

En outre, une composition cosmétique contient le plus souvent un ou plusieurs actifs. Mais ce type de vésicules limite aussi la concentration de certains actifs. Par exemple, la glycérine ne peut être utilisée qu'en quantité limitée afin que la texture ne soit pas collante.

Il subsiste donc le besoin de dispersions permettant de remédier à ces inconvénients et agréables à utiliser quels que soient les composés qu'elles contiennent.

La demanderesse a trouvé, de façon inattendue, des vésicules permettant d'atteindre ces objectifs.

En effet, la demanderesse a trouvé que l'on pouvait obtenir une dispersion de vésicules donnant une texture non collante, sans être limité dans le choix et/ou la concentration des huiles, des actifs ou des gélifiants de la dispersion, en utilisant des vésicules formées d'un tensioactif siliconé.

Aussi, la présente invention a pour objet une dispersion dans une phase aqueuse externe, d'au moins une phase liquide non miscible à l'eau à l'aide de vésicules lipidiques encapsulant une phase interne, caractérisée en ce que les vésicules lipidiques comportent une phase lamellaire formée d'au moins un tensioactif siliconé.

La phase interne est généralement une phase aqueuse.

Un tensioactif siliconé est un composé siliconé comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5364633 et US-A-5411744. Ces documents décrivent l'utilisation de tensioactifs siliconés pour préparer des vésicules lipidiques. Toutefois, ils ne décrivent ni ne suggèrent que ces vésicules puissent assurer la dispersion, dans une phase aqueuse, d'un liquide non miscible à l'eau. Or, il n'était pas évident, étant donné la nature chimique particulière de ces tensioactifs, que la phase lamellaire les contenant puisse assurer une bonne dispersion d'une phase non miscible à l'eau dans une phase aqueuse sans ajout d'autre agent de stabilisation et notamment d'émulsionnant.

Par ailleurs, il est connu par les documents EP-A-444983 et EP-A-526289 de préparer des vésicules lipidiques contenant un lipide ionique ou non-ionique associé à un composé siliconé dans la phase lamellaire et d'incorporer ces vésicules dans une dispersion aqueuse pouvant contenir de l'huile. Mais le composé siliconé décrit dans ces documents ne forme pas l'élément constitutif des vésicules ; il ne constitue qu'un additif qui s'intercale entre les feuillets formés par le lipide, mais ne forme pas ces feuillets. En effet, il s'agit d'une huile, d'une gomme ou d'une résine de silicone, dont la constitution chimique ne leur permet pas de former des vésicules. En revanche, dans la présente invention, le tensioactif siliconé est l'élément essentiel constitutif des vésicules.

Il est également connu par EP-A-724876 une méthode d'encapsulation d'une substance hydrosoluble ou liposoluble (PDMS, huile de jojoba, huile minérale...) dans des vésicules formés à partir de tensioactifs de type siloxane.

De manière avantageuse, le tensioactif siliconé utilisé selon l'invention est un composé de formule (I) : 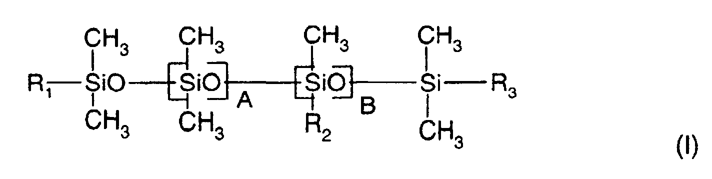 dans laquelle
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation préféré de l'invention, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) : 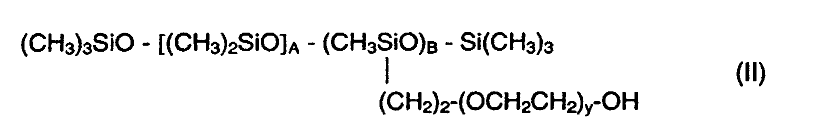 dans laquelle A est un nombre entier allant de 20 à 105, B est un nombre entier allant de 2 à 10 et y est un nombre entier allant de 10 à 20.

On peut également citer à titre d'exemple de tensioactifs siliconés de formule (I), les composés de formule (III) :

HO-(CH₂CH₂O)_{y}-(CH₂)₃-[(CH₃)₂SiO]_{A'}-Si(CH₃)₂-(CH₂)₃-(OCH₂CH₂)_{y}-OH (III)

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le composé Q4-3667 est un composé de formule (III) où A' est 15 et y est 13.

Le tensioactif siliconé est présent dans la dispersion en une quantité allant de 0,1 à 40 %, et de préférence de 1 à 10 % en poids par rapport au poids total de la dispersion.

Les dispersions obtenues avec les vésicules selon l'invention ne sont pas collantes à l'application sur la peau et/ou les cheveux contrairement aux dispersions de l'état de la technique. En outre, elles ont une texture très douce, fluide et légère, et sont de ce fait particulièrement intéressantes comme compositions pour le traitement des peaux grasses.

On ajoute, en outre, un lipide amphiphile ionique au tensioactif siliconé formant les vésicules. L'addition d'un tel lipide améliore la stabilité de la dispersion selon l'invention par inhibition de la floculation.

Les lipides amphiphiles ioniques utilisés selon l'invention peuvent être choisis notamment dans le groupe formé par les lipides anioniques neutralisés, les lipides ioniques amphotères, les dérivés alkylsulfoniques et leurs mélanges.

Ils sont plus particulèrement choisis dans le groupe formé par :
- les sels alcalins du dicetyl- et du dimyristylphosphate ;
- les sels alcalins du cholestérol sulfate ;
- les sels alcalins du cholestérol phosphate ;
- les lipoaminoacides tels que les acylglutamates mono et disodiques ;
- les sels de sodium de l'acide phosphatidique ;
- les phospholipides ;
- les dérivés alkylsulfoniques de formule : 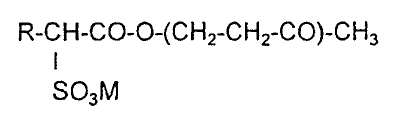
dans laquelle R représente des radicaux alkyle en C₁₆-C₂₂, en particulier les radicaux C₁₆H₃₃ et C₁₈H₃₇, pris en mélange ou séparément, et M est un métal alcalin tel que le sodium et le potassium ; - et leurs mélanges.

Comme lipide amphiphile ionique, on peut citer en particulier le sel monosodique de l'acide N-stéaroylglutamique vendu sous la dénomination « acylglutamate HS 21 » par la société Ajinomoto, le dicétylphosphate de sodium et le dimyristylphosphate de sodium.

Les lipides ioniques amphiphiles sont en particulier présents à des concentrations allant jusqu'à 20 % en poids, plus particulièrement de 5 à 10 % en poids par rapport au poids de tensioactif siliconé.

On peut aussi ajouter au tensioactif siliconé formant les vésicules, au moins un additif permettant de diminuer la perméabilité des vésicules, de prévenir leur fusion et d'augmenter le taux d'encapsulation. L'additif peut être notamment choisi dans le groupe formé par les stérols et notamment les phytostérols et le cholestérol, les alcools et diols à longue chaîne, les amines à longue chaîne et leurs dérivés ammonium quaternaire, et leurs mélanges.

Cet additif peut avoir une activité cosmétique et/ou dermatologique ; c'est le cas, par exemple, du cholestérol.

Les additifs sont en particulier présents en des concentrations allant de 0 à 50 % en poids, plus particulièrement de 5 à 40 % en poids par rapport au poids de tensioactif siliconé.

Selon l'invention, le liquide non miscible à l'eau est une huile ou un mélange d'huiles, pouvant contenir aussi d'autres matières grasses telles que des alcools gras, des acides gras, des cires, des gommes.

Comme huiles utilisables dans l'invention, on peut citer les huiles minérales, les huiles végétales (huile de jojoba), les huiles animales, les huiles de synthèse, les huiles essentielles naturelles ou synthétiques, les huiles siliconées (huile de silicone volatile), les huiles fluorées (perfluoropolyéthers), les carbures halogénés.

Les vésicules selon l'invention peuvent contenir, de façon connue, un ou plusieurs composés actifs, notamment les actifs cosmétiques et/ou dermatologiques qui, selon leurs caractéristiques de solubilité, peuvent avoir différentes localisations. Si les actifs sont hydrosolubles, ils se trouvent dans la phase aqueuse encapsulée des vésicules. Si les actifs sont liposolubles, ils se trouvent dans la phase lipidique constituant la phase lamellaire. Si les actifs sont amphiphiles, ils se répartissent entre la phase lipidique et la phase aqueuse encapsulée avec un coefficient de partage qui varie selon la nature de l'actif amphiphile et les compositions respectives de la phase lipidique et de la phase aqueuse encapsulée.

Les actifs peuvent se trouver également dans la phase aqueuse externe et/ou dans le liquide non miscible à l'eau.

Ces actifs peuvent être, entre autres, des émollients, des humectants, des agents antiradicalaires, des agents anti-oxydants, des agents anti-inflammatoires, des vitamines, des agents dépigmentants, des agents anti-acnéiques, des agents antiséborrhéiques, des agents kératolytiques, des amincissants, des agents de coloration de la peau, des filtres solaires, des huiles essentielles, des pigments, des agents accélérateurs de bronzage, des parfums, des colorants, des agents mélanorégulateurs, des agents anti-rides et anti-vieillissement, des agents liporégulateurs, des agents antibactériens, des agents antifongiques, des antiperspirants, des déodorants, des agents immunomodulateurs, des agents cicatrisants, des agents protecteurs vasculaires, des agents conditionneurs de la peau.

Ces actifs sont utilisés dans les quantités habituelles dans les domaines concernés.

Comme actifs contenus dans les vésicules selon l'invention, on peut citer par exemple les polyols tels que la glycérine.

La phase aqueuse de la dispersion peut contenir des adjuvants cosmétiquement et/ou dermatologiquement acceptables. Parmi ces adjuvants, on peut citer les conservateurs, les gélifiants (carbomer) et épaississants, les matières colorantes, les parfums, les opacifiants.

La dispersion selon l'invention peut constituer notamment une composition topique, en particulier cosmétique et/ou dermatologique. Pour une application topique, la dispersion selon l'invention contient un milieu topiquement acceptable, c'est-à-dire compatible avec la peau, les muqueuses, les ongles, le cuir chevelu et les cheveux.

L'invention a aussi pour objet l'utilisation de la dispersion définie ci-dessus pour le traitement cosmétique de la peau et/ou les muqueuses et/ou des ongles et/ou du cuir chevelu et/ou des cheveux ainsi que pour la préparation d'une composition destinée au traitement dermatologique des maladies de la peau et/ou les muqueuses et/ou des ongles et/ou du cuir chevelu et/ou des cheveux.

L'invention a encore pour objet un procédé de traitement non-thérapeutique et/ou thérapeutique de la peau et/ou des muqueuses et/ou les ongles et/ou du cuir chevelu et/ou des cheveux consistant à appliquer la dispersion définie ci-dessus sur la peau et/ou les muqueuses et/ou les ongles et/ou le cuir chevelu et/ou les cheveux.

La dispersion selon l'invention permet en particulier le traitement des peaux grasses. Aussi, la présente invention se rapporte également à l'utilisation de la dispersion définie ci-dessus pour traiter la peau grasse.

La dispersion selon l'invention peut se présenter sous toutes les formes galéniques normalement utilisées pour une application topique, et peut constituer notamment une crème, un lait ou un sérum.

L'exemple ci-après de composition selon l'invention, est donné à titre d'illustration et sans caractère limitatif. Les quantités y sont données en % en poids.

### Exemple : Crème de jour pour peaux grasses

*Première phase :*
- Tensioactif siliconé (DC 2-5695) 5 %
- Acylglutamate HS 21 0,6 %
- Glycérine 3 %
- Eau déminéralisée 60,6 %

*Deuxième phase :*
- Huile de silicone volatile 10 %
- Huile de jojoba 10 %
- Carbomer (Carbopol 980 vendu par la société Goodrich) 0,42 %
- Conservateurs 0,3 %
- Triéthanolamine qsp pH = 6
- Eau déminéralisée qsp 100 %

Pour préparer la crème, on a suivi le mode opératoire suivant : on a mélangé le tensioactif siliconé avec l'acylglutamate, puis on a préparé la première phase en hydratant progressivement ce mélange avec le mélange d'eau et de glycérine, et en l'homogénéisant à l'aide d'un mélangeur classique (Moritz). On a obtenu une suspension de vésicules dont la taille est de l'ordre de 200 nm. On a dispersé ensuite la phase huileuse dans la suspension de vésicules et on a effectué deux passages du mélange à l'homogénéisateur haute pression à 500 bars. Puis, on a ajouté le gel de Carbopol neutralisé préalablement préparé.

Cette crème est une crème blanche, de texture fine et non collante, agréable à utiliser.

## Revendications

1. Dispersion dans une phase aqueuse externe, d'au moins une phase liquide non miscible à l'eau qui est une huile ou un mélange d'huiles pouvant contenir des alcools gras, des acides gras, des cires, des gommes, à l'aide de vésicules lipidiques encapsulant une phase interne et comportant une phase lamellaire formée d'au moins un tensioactif siliconé, ladite phase lamellaire comprenant en outre au moins un lipide amphiphile ionique.

2. Dispersion selon la revendication 1, **caractérisée en ce que** la phase interne est une phase aqueuse.

3. Dispersion selon la revendication 1 ou 2, **caractérisée en ce que** le tensioactif siliconé est un composé de formule (I) : 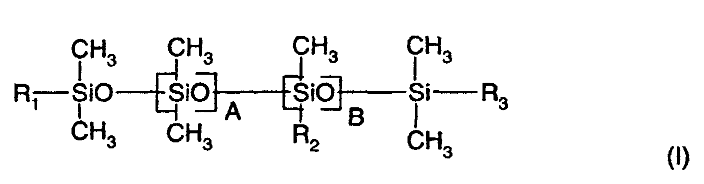 dans laquelle :
R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, au moins un radical R₁, R₂ ou R₃ n'étant pas un radical alkyle ; R₄ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

4. Dispersion selon la revendication 3, **caractérisée en ce que** le tensioactif siliconé est un composé de formule (I) où le radical alkyle est un radical méthyle, x est un nombre entier allant de 2 à 6 et y est un nombre entier allant de 4 à 30.

5. Dispersion selon l'une quelconque des revendications précédentes, **caractérisée en ce que** le tensioactif siliconé est un composé de formule (II) : 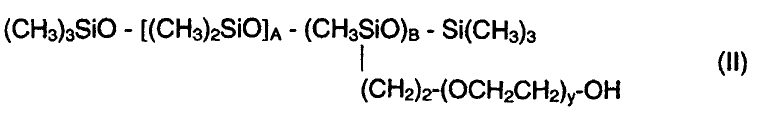 dans laquelle A est un nombre entier allant de 20 à 105, B est un nombre entier allant de 2 à 10 et y est un nombre entier allant de 10 à 20.

6. Dispersion selon la revendication précédente, **caractérisée en ce que** le tensioactif siliconé est choisi parmi les composés de formule (II) où A est 22, B est 2 et y est 12 ; A est 103, B est 10 et y est 12 ; A est 27, B est 3 et y est 12.

7. Dispersion selon l'une quelconque des revendications précédentes, **caractérisée en ce que** le tensioactif siliconé est présent en une quantité allant de 0,1 à 40 % en poids par rapport au poids total de la dispersion.

8. Dispersion selon l'une quelconque des revendications précédentes, **caractérisée en ce que** le tensioactif siliconé est présent en une quantité allant de 1 à 10 % en poids par rapport au poids total de la dispersion.

9. Dispersion selon l'une des revendications précédentes, **caractérisée en ce que** le lipide amphiphile ionique est choisi dans le groupe formé par les lipides anioniques neutralisés, les lipides ioniques amphotères, les dérivés alkylsulfoniques et leurs mélanges.

10. Dispersion selon l'une des revendications précédentes, **caractérisée en ce que** le lipide amphiphile ionique est choisi dans le groupe formé par :
- les sels alcalins du dicetyl- et du dimyristylphosphate ;
- les sels alcalins du cholestérol sulfate ;
- les sels alcalins du cholestérol phosphate ;
- les sels de lipoaminoacides ;
- les sels de sodium de l'acide phosphatidique ;
- les phospholipides ;
- les dérivés alkylsulfoniques de formule : 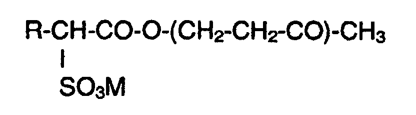 dans laquelle R représente des radicaux alkyle en C₁₆-C₂₂ pris en mélange ou séparément et M est un métal alcalin ;
- et leurs mélanges.

11. Dispersion selon l'une quelconque des revendications précédentes, **caractérisée en ce que** le lipide amphiphile ionique est présent en une concentration allant de 5 à 10 % en poids par rapport au poids de tensioactif siliconé.

12. Dispersion selon l'une quelconque des revendications précédentes,
**caractérisée en ce que** la phase lamellaire comprend, en outre, au moins un additif choisi dans le groupe formé par les stérols, les alcools et diols à longue chaîne, les amines à longue chaîne et leurs dérivés ammonium quaternaire, et leurs mélanges.

13. Dispersion selon la revendication précédente, **caractérisée en ce que** l'additif est le cholestérol ou un phytostérol.

14. Dispersion selon l'une des revendications 12 à 13, **caractérisée en ce que** l'additif est présent en une concentration allant de 5 à 40 % en poids par rapport au poids de tensioactif siliconé.

15. Dispersion selon l'une quelconque des revendications précédentes, **caractérisée en ce que** la phase interne des vésicules contient au moins un actif cosmétique ou dermatologique.

16. Dispersion selon l'une quelconque des revendications précédentes, **caractérisée en ce que** le liquide non miscible à l'eau est une huile.

17. Dispersion selon l'une quelconque des revendications précédentes, **caractérisée en ce qu'**elle consiste en une composition cosmétique et/ou dermatologique.

18. Utilisation non thérapeutique de la dispersion selon l'une quelconque des revendications précédentes pour le traitement cosmétique de la peau et/ou des muqueuses et/ou des ongles et/ou du cuir chevelu et/ou des cheveux.

19. Utilisation selon la revendication 18, pour traiter la peau grasse.

20. Utilisation de la dispersion selon l'une quelconque des revendications 1 à 17 pour la préparation d'une composition destinée au traitement dermatologique des maladies de la peau et/ou des muqueuses et/ou des ongles et/ou du cuir chevelu et/ou des cheveux.

21. Procédé de traitement non-thérapeutique de la peau et/ou des muqueuses et/ou les ongles et/ou du cuir chevelu et/ou des cheveux, **caractérisé en ce qu'**il consiste à appliquer sur la peau et/ou les muqueuses et/ou les ongles et/ou le cuir chevelu et/ou les cheveux, la dispersion selon l'une quelconque des revendications 1 à 17.

## Claims

1. Dispersion, in an outer aqueous phase, of at least one water-immiscible liquid phase which is an oil or a mixture of oils possibly containing fatty alcohols, fatty acids, waxes or gums, using lipid vesicles encapsulating an inner phase and including a lamellar phase formed from at least one silicone surfactant, the said lamellar phase also comprising at least one ionic amphiphilic lipid.

2. Dispersion according to Claim 1, **characterized in that** the inner phase is an aqueous phase.

3. Dispersion according to Claim 1 or 2, **characterized in that** the silicone surfactant is a compound of formula (I) : 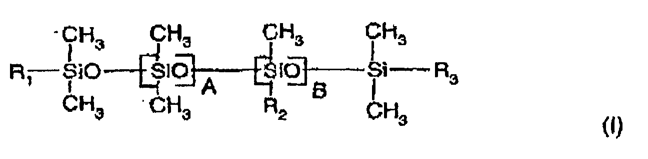 in which:
R₁, R₂ and R₃, independently of each other, represent a C₁-C₆ alkyl radical or a radical -(CH₂)ₓ-(OCH₂CH₂)_{y}-(OCH₂CH₂CH₂)_{z}-OR₄, at least one radical R₁, R₂ or R₃ not being an alkyl radical; R₄ being a hydrogen, an alkyl radical or an acyl radical;
A is an integer ranging from 0 to 200;
B is an integer ranging from 0 to 50; on condition that A and B are not simultaneously equal to zero;
x is an integer ranging from 1 to 6;
y is an integer ranging from 1 to 30;
z is an integer ranging from 0 to 5.

4. Dispersion according to Claim 3, **characterized in that** the silicone surfactant is a compound of formula (I) in which the alkyl radical is a methyl radical, x is an integer ranging from 2 to 6 and y is an integer ranging from 4 to 30.

5. Dispersion according to any one of the preceding claims, **characterized in that** the silicone surfactant is a compound of formula (II) : 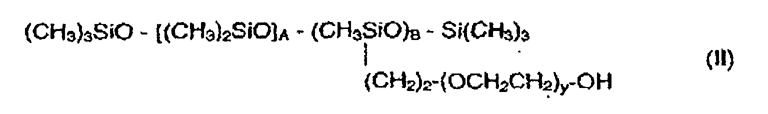 in which A is an integer ranging from 20 to 105, B is an integer ranging from 2 to 10 and y is an integer ranging from 10 to 20.

6. Dispersion according to the preceding claim, **characterized in that** the silicone surfactant is chosen from the compounds of formula (II) in which A is 22, B is 2 and y is 12; A is 103, B is 10 and y is 12; A is 27, B is 3 and y is 12.

7. Dispersion according to any one of the preceding claims, **characterized in that** the silicone surfactant is present in an amount ranging from 0.1% to 40% by weight relative to the total weight of the dispersion.

8. Dispersion according to any one of the preceding claims, **characterized in that** the silicone surfactant is present in an amount ranging from 1% to 10% by weight relative to the total weight of the dispersion.

9. Dispersion according to one of the preceding claims, **characterized in that** the ionic amphiphilic lipid is chosen from the group formed by neutralized anionic lipids, amphoteric ionic lipids and alkylsulphonic derivatives, and mixtures thereof.

10. Dispersion according to one of the preceding claims, **characterized in that** the ionic amphiphilic lipid is chosen from the group formed by:
- alkali metal salts of dicetyl phosphate and of dimyristyl phosphate;
- alkali metal salts of cholesteryl sulphate;
- alkali metal salts of cholesteryl phosphate;
- lipoamino acid salts;
- the sodium salts of phosphatidic acid;
- phospholipids;
- alkylsulphonic derivatives of formula: 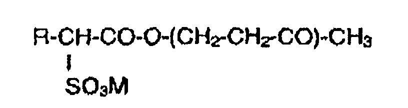 in which R represents C₁₆-C₂₂ alkyl radicals taken as a mixture or separately, and M is an alkali metal;
- and mixtures thereof.

11. Dispersion according to any one of the preceding claims, **characterized in that** the ionic amphiphilic lipid is present in a concentration ranging from 5% to 10% by weight relative to the total weight of silicone surfactant.

12. Dispersion according to any one of the preceding claims, **characterized in that** the lamellar phase also comprises at least one additive chosen from the group formed by sterols, long-chain alcohols and diols, long-chain amines and quaternary ammonium derivatives thereof, and mixtures thereof.

13. Dispersion according to the preceding claim, **characterized in that** the additive is cholesterol or a phytosterol.

14. Dispersion according to either of Claims 12 and 13, **characterized in that** the additive is present in a concentration ranging from 5% to 40% by weight relative to the weight of silicone surfactant.

15. Dispersion according to any one of the preceding claims, **characterized in that** the inner phase of the vesicles contains at least one cosmetic or dermatological active agent.

16. Dispersion according to any one of the preceding claims, **characterized in that** the water-immiscible liquid is an oil.

17. Dispersion according to any one of the preceding claims, **characterized in that** it consists of a cosmetic and/or dermatological composition.

18. Non-therapeutic use of the dispersion according to any one of the preceding claims, for the cosmetic treatment of the skin and/or mucous membranes and/or the nails and/or the scalp and/or the hair.

19. Use according to Claim 18, for treating greasy skin.

20. Use of the dispersion according to any one of Claims 1 to 17, for the preparation of a composition intended for the dermatological treatment of diseases of the skin and/or of mucous membranes and/or of the nails and/or of the scalp and/or of the hair.

21. Non-therapeutic process for treating the skin and/or mucous membranes and/or the nails and/or the scalp and/or the hair, **characterized in that** it consists in applying to the skin and/or mucous membranes and/or the nails and/or the scalp and/or the hair the dispersion according to any one of Claims 1 to 17.

## Patentansprüche

1. Dispersion mindestens einer nicht mit Wasser mischbaren, flüssigen Phase, bei der es sich um ein Öl oder ein Gemisch von Ölen handelt, die Fettalkohole, Fettsäuren, Wachse und Gummis/Gummen enthalten können, in einer äußeren wässerigen Phase unter Verwendung von Lipidvesikeln, die eine innere Phase einkapseln und die eine lamellare Phase enthalten, die aus mindestens einem siliconhaltigen grenzflächenaktiven Stoff gebildet wird, wobei die lamellare Phase ferner mindestens ein ionisches amphiphiles Lipid enthält.

2. Dispersion nach Anspruch 1, **dadurch gekennzeichnet, daß** die innere Phase eine wässerige Phase ist.

3. Dispersion nach Anspruch 1 oder 2, **dadurch gekennzeichnet, daß** der siliconhaltige grenzflächenaktive Stoff eine Verbindung der Formel (I) ist: 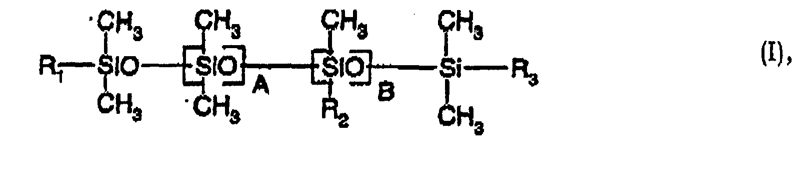 worin bedeuten:
- R₁, R₂ und R₃ unabhängig voneinander eine C₁₋₆-Alkylgruppe oder eine Gruppe -(CH₂)ₓ-(OCH₂CH₂)_{y}-(OCH₂CH₂CH₂)_{z}-OR₄, wobei mindestens eine Gruppe R₁, R₂ oder R₃ keine Alkylgruppe ist und R₄ Wasserstoff, eine Alkylgruppe oder eine Acylgruppe bedeutet,
- A Null oder eine ganze Zahl von 1 bis 200,
- B Null oder eine ganze Zahl von 1 bis 50, mit der Maßgabe, daß A und B nicht gleichzeitig Null bedeuten,
- x eine ganze Zahl von 1 bis 6,
- y eine ganze Zahl von 1 bis 30,
und
- z Null oder eine ganze Zahl von 1 bis 5.

4. Dispersion nach Anspruch 3, **dadurch gekennzeichnet, daß** der siliconhaltige grenzflächenaktive Stoff eine Verbindung der Formel (I) ist, in der die Alkylgruppe eine Methylgruppe, x eine ganze Zahl von 2 bis 6 und y eine ganze Zahl von 4 bis 30 bedeutet.

5. Dispersion nach einem der vorhergehenden Ansprüche, **dadurch gekennzeichnet, daß** der siliconhaltige grenzflächenaktive Stoff eine Verbindung der Formel (II) ist: 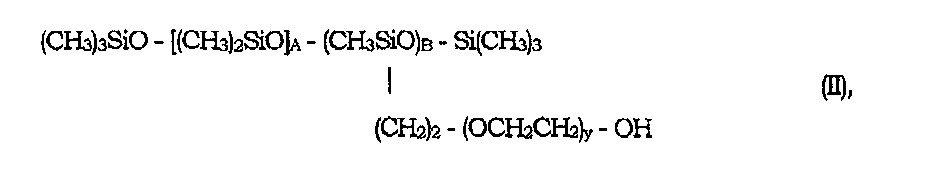 worin bedeuten: A eine ganze Zahl von 20 bis 105, B eine ganze Zahl von 2 bis 10 und y eine ganze Zahl von 10 bis 20.

6. Dispersion nach dem vorhergehenden Anspruch, **dadurch gekennzeichnet, daß** der siliconhaltige grenzflächenaktive Stoff unter den Verbindungen der Formel (II) ausgewählt ist, in der A 22, B 2 und y 12 bzw. A 103, B 10 und y 12 bzw. A 27, B 3 und y 12 bedeuten.

7. Dispersion nach einem der vorhergehenden Ansprüche, **dadurch gekennzeichnet, daß** der siliconhaltige grenzflächenaktive Stoff in einem Mengenanteil von 0,1 bis 40 Gew.-%, bezogen auf das Gesamtgewicht der Dispersion, vorliegt.

8. Dispersion nach einem der vorhergehenden Ansprüche, **dadurch gekennzeichnet, daß** der siliconhaltige grenzflächenaktive Stoff in einem Mengenanteil von 1 bis 10 Gew.-%, bezogen auf das Gesamtgewicht der Dispersion, vorliegt.

9. Dispersion nach einem der vorhergehenden Ansprüche, **dadurch gekennzeichnet, daß** das ionische amphiphile Lipid unter den neutralisierten anionischen Lipiden, den amphoteren ionischen Lipiden und den Alkylsulfonsäurederivaten und deren Gemischen ausgewählt ist.

10. Dispersion nach einem der vorhergehenden Ansprüche, **dadurch gekennzeichnet, daß** das ionische amphiphile Lipid ausgewählt ist unter:
- den Alkalisalzen von Dicetyl- und Dimyristylphosphat;
- den Alkalisalzen von Cholesterinsulfat;
- den Alkalisalzen von Cholesterinphosphat;
- den Lipoaminosäuren;
- den Natriumsalzen von Phosphatidsäure;
- den Phospholipiden;
- den Alkylsulfonsäurederivaten der Formel: 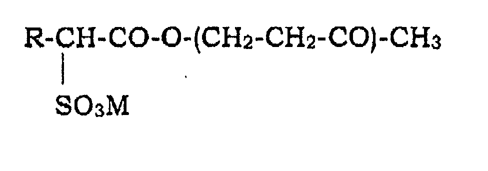 worin R eine Alkylgruppe mit 16 bis 22 Kohlenstoffatomen als Gemisch oder einzeln bedeutet und M ein Alkalimetall ist; und
- deren Gemischen.

11. Dispersion nach einem der vorhergehenden Ansprüche, **dadurch gekennzeichnet, daß** das ionische amphiphile Lipid in einer Konzentration von 5 bis 10 Gew.-%, bezogen auf das Gewicht des grenzflächenaktiven Silicons, vorliegt.

12. Dispersion nach einem der vorhergehenden Ansprüche, **dadurch gekennzeichnet, daß** die lamellare Phase ferner mindestens einen Zusatz enthält, der unter den Sterinen, den langkettigen Alkoholen und Diolen, den langkettigen Aminen und ihren quartären Ammoniumderivaten und ihren Gemischen ausgewählt ist.

13. Dispersion nach dem vorhergehenden Anspruch, **dadurch gekennzeichnet, daß** der Zusatz das Cholesterin oder ein Phytosterin ist.

14. Dispersion nach einem der Ansprüche 12 oder 13, **dadurch gekennzeichnet, daß** der Zusatz in einer Konzentration von 5 bis 40 Gew.-%, bezogen auf das Gewicht des grenzflächenaktiven Silicons, vorliegt.

15. Dispersion nach einem der vorhergehenden Ansprüche, **dadurch gekennzeichnet, daß** die innere Phase der Vesikel mindestens einen kosmetischen oder dermatologischen Wirkstoff enthält.

16. Dispersion nach einem der vorhergehenden Ansprüche, **dadurch gekennzeichnet, daß** die nicht mit Wasser mischbare Flüssigkeit ein Öl ist.

17. Dispersion nach einem der vorhergehenden Ansprüche, **dadurch gekennzeichnet, daß** es sich um eine kosmetische und/oder dermatologische Zusammensetzung handelt.

18. Nicht therapeutische Verwendung der Dispersion nach einem der vorhergehenden Ansprüche zur kosmetischen Behandlung der Haut und/oder der Schleimhäute und/oder der Nägel und/oder der Kopfhaut und/oder der Haare.

19. Verwendung nach Anspruch 18 zur Behandlung von fettiger Haut.

20. Verwendung der Dispersion nach einem der Ansprüche 1 bis 17 zur Herstellung von Zusammensetzungen, die zur dermatologischen Behandlung von Erkrankungen der Haut und/oder der Schleimhäute und/oder der Nägel und/oder der Kopfhaut und/oder der Haare vorgesehen sind.

21. Verfahren zur nicht therapeutischen Behandlung der Haut und/oder der Schleimhäute und/oder der Nägel und/oder der Kopfhaut und/oder der Haare, **dadurch gekennzeichnet, daß** es darin besteht, die Dispersion nach einem der Ansprüche 1 bis 17 auf die Haut und/oder die Schleimhäute und/oder die Nägel und/oder die Kopfhaut und/oder die Haare aufzutragen.
